# EUROPEAN PATENT SPECIFICATION

(11) **EP 2 802 873 B1**
(45) Date of publication and mention of the grant of the patent: **31.05.2017**
(21) Application number: 13723662.6
(22) Date of filing: 11.01.2013
(51) Int. Cl.: G01N 33/487

(54) **METHOD AND CONTAINER FOR LOADING A TEST STRIP METER WITH A SINGLE TEST STRIP**
VERFAHREN UND BEHÄLTER ZUR BELASTUNG EINES TESTSTREIFEN-MESSGERÄT MIT EINEM EINZIGEN TESTSTREIFEN
PROCÉDÉ ET CONTENANT PERMETTANT DE CHARGER UN DISPOSITIF DE MESURE DE BANDELETTES AVEC UNE BANDELETTE INDIVIDUELLE

(30) Priority: 18.01.2012 EP 12000258
(43) Date of publication of application: 19.11.2014
(73) Proprietor: Medical Device UG, 59199 Bönen (DE)
(72) Inventor: BARTETZKO, Norbert, 59071 Hamm (DE); BARTETZKO, Robert, 59071 Hamm (DE)
(74) Representative: Leifert & Steffan
(86) International application number: PCT/EP2013/000064
(87) International publication number: WO 2013/117287

(56) References cited:
- US-A- 5 736 103
- US-A1- 2003 212 345
- US-A1- 2009 151 448

## Description

The present invention refers to a method and a container for loading a measuring device with measuring means units. In particular, the invention refers to a container for a plurality of measuring means units in form of test strips to be loaded into a blood glucose monitoring device for measuring the blood glucose level of a user by using one of the test strips.

The monitoring of the blood glucose level is an essential everyday commodity in the life of many diabetics. Correct measuring results and a repeated testing everyday are important for a symptom-free life with diabetes. A wrong behaviour of a diabetic may have severe consequences, such as cardiovascular diseases, damages in the nervous system or blindness.

Most portable measuring devices, with which a user can measure independently his/her own blood glucose level, are based on an amperometric determination of the blood glucose concentration. For this, a test strip comprises an absorptive portion for receiving a drop of a body fluid, such as blood or tears, wherein the body fluid is transported into a sensor portion through a capillary. In the sensor portion, the blood fluid reacts by means of the glucose oxidase enzyme and closes an electric circuit between two electrodes within the test strip.

Each electrode is connected in an electrically conducting way with a contact surface, both contact surfaces being located at one end of the test strip. Before a measurement, said end of the test strip being equipped with the contact surfaces is inserted into an adequate measuring device, where contact pins touch on the contact surfaces. Having applied a drop of body fluid on the absorptive portion of the test strip, wherein the absorptive portion protrudes out of the measuring device, a current progression can be measured by the measuring device applying a voltage to the electrodes, the current progression being characteristic for the blood glucose concentration. A test strip is consumed after one measurement, such that for each measurement a new, dry and clean test strip must be used.

Having a closer look at the different blood glucose monitoring device, it becomes apparent that they differ in many details indeed, but may in principle be divided into two groups: capsule systems and integrated systems.

Using capsule systems, test strips are stored in a capsule separate from the measuring device. A user retrieves manually a test strip and puts it also manually into the measuring device.

Documents EP 1 352 611 B1, EP 1 854 738 B1, US 6,508,380 B1 and WO 2008/125244 A1 describe dispenser capsules designed for storing and dispensing test strips. In those systems, the uppermost test strip is pushed out of a dispenser opening such that it can be grabbed with the fingers and pulled out completely. Afterwards, the test strip is inserted manually into the measuring device.

It is an advantage of those systems that the measuring device can be designed very compact. However, it is a disadvantage that the manual loading of the measuring device is unhygienic, because the test strip to be inserted may be contaminated by touching it with the hands and, as the case may be, it may absorb moisture which may impair the measuring result. On the other hand, a dedicated touching section at the test strip yields test strips with an undesired large size. Furthermore, many diabetics are very old and have a limited dexterity such that a manual loading is often impossible. Even for users with a good dexterity the test strips must be designed quite large for the loading to be user-friendly. On the market, a minimum test strip length of 27 mm has been established. However, such a minimum length is not necessary to ensure a reliable measurement according to German Industry Standard DIN EN ISO 15197.

In so-called "integrated systems", a test strip container in form of a cylinder or a cartridge is integrated or insertable into the measuring device. These systems are more hygienic and may be loaded with smaller test strips. There is no preparation needed before each measurement and there is no need to carry individual components. When necessary, the user merely needs to exchange an emptied cylinder or cartridge by a filled one. However, it is a disadvantage of integrated systems that they need a relatively complex mechanism for loading the measuring device with a test strip, and that they are therefore for example too big and heavy to take-away out and about. Furthermore, the complex mechanisms are prone to malfunctions.

Document EP 2 157 428 A1 describes a dispenser that can be operated in a "stand-alone" mode and an "attached-to-meter" mode. In the "stand-alone" mode, the dispenser dispenses a test strip for the ordinary manual retrieval. In the "attached-to-meter" mode, the dispenser dispenses a test strip upon manual shifting a slide button after docking the dispenser to the measuring device and inserts the test strip into the measuring device by the slide button. This system has the disadvantage that it is awkward or even completely impossible with limited dexterity to manually shift the slide button while keeping the dispenser docked to the measuring device.

Furthermore, US 2009/0151448 A1 discloses a test piece magazine holder and test piece attachment apparatus. US 2003/0212345 A1 is directed to a minimal procedure analyte test system. US patent no. 5,736,103 discloses a remote-dosing analyte concentration meter.

It is therefore the object of the present invention to provide a method and a container for loading a measuring device with a measuring means unit such that the loading is more hygienic, more economically efficient, and easier to perform.

This object is solved by the method and container according to claims 1 and 6, respectively, as well as a kit according to claim 13. Advantageous embodiments of the invention may are described in the corresponding dependent claims, the description, or the figures.

According to a first aspect of the invention, a method for loading a measuring device with a measuring means unit is provided, the method comprising the steps of:
- inserting at least one portion of the measuring device into a container, said container comprising a plurality of measuring means units;
- retrieving one of the measuring means units inside of the container by means of the measuring device; and
- retracting at least one portion of the measuring device together with the retrieved measuring means unit out of the container,
and making use of the kit of any of claims 13 to 15.

The measuring device is thus inserted with at least one portion of it, i.e. preferably with an insertion side of the measuring device, into the container and retrieves, inside of the container, one of the measuring means units stored in the container, and is retracted out of the container together with the retrieved measuring means unit. Hence, said container is, unlike the prior art, no dispenser, because it does not dispense any measuring means units. In contrast, according to the present invention, the measuring device enters into the container to take out a measuring means unit.

The first aspect of the invention is directed to a method for loading a blood glucose level measuring device with a blood glucose level test strip, the method making use of the kit of any of claims 13 to 15 and comprising the steps of:
- inserting at least one portion of the measuring device into a container, said container comprising a plurality of test strips;
- retrieving one of the test strips inside of the container by means of the measuring device; and retracting at least one portion of the measuring device together with the retrieved test strip out of the container.

Furthermore, it should be noted that the method is not restricted to embodiments where the container is stationary and the measuring device is exclusively moving towards (away from) the container. The step of inserting (retracting) of at least one portion of the measuring device should be understood as relative movement between the measuring device and the container, such that both the measuring device and the container may be moving towards (away from) each other, or the container may be moving away from (towards) the measuring device, or the measuring device may be moving towards (away from) the container.

The method according to the present invention combines the advantage of a compact measuring device of capsule systems and the hygienic and safe loading of integrated systems. Compared to EP 2 157 428 A1, the invention has the advantage that a relatively small measuring means unit can be loaded into the measuring device by a physically very simple manual operation without any additional operation of a slide button.

Preferably, the container is separate from the measuring device and can be manually brought together with the measuring device. However, a mechanical coupling between the measuring device and the container by means of guidance, e.g. a rail, is also conceivable. Bringing together container and measuring device plus the steps of inserting and retracting are preferably performed manually, wherein one hand holds the container and other hand holds the measuring device. The hands must simply be moved towards each other and away from each other. A sound dexterity is not needed for this.

Preferably, the method further comprises the steps of:
- displacing a plurality of measuring means units within the container by the step of inserting the at least one portion of the measuring device against a first resilient force, wherein the measuring means units are located inside the container; and
- holding in a retrieval position exactly one of the measuring means units being located inside the container, such that said measuring means unit is not displaced during the step of displacing of the other measuring means units and such that it is retrieved in the retrieval position by means of the measuring device.

Therein, the retrieval position is preferably defined by means of the container, such that a retrieval means being arranged at the at least one portion of the measuring device reaches exactly into the retrieval position after the step of inserting. Through the step of displacing the other measuring means units, the exactly one measuring means unit being held in the retrieval position is separated from the other measuring means units and can preferably be jammed in the retrieving means of the measuring device

Preferably, the displaced measuring means units being located inside the container are further displaced back to an initial configuration within the container by the first resilient force during the step of retracting the at least one portion of the measuring device. Thereby, a new initial configuration for a subsequent loading is achieved in a simple way.

Preferably, the displaced measuring means units are further shifted within the container by a second resilient force, such that one of the measuring means units not being retrieved is shifted into the retrieval position Thereby, the next measuring means unit is positioned in a simple way into the retrieval position for a subsequent loading.

Preferably, the shifting is carried out essentially orthogonally with respect to the displacing. This is of particular advantage if the container comprises a stack of measuring means units having the form of strips within a magazine unit. The first resilient force may preferably move the magazine unit itself within the container along a first axis. Along this first axis, the measuring device is preferably inserted into the container. Along a second axis orthogonal to the first axis, the second resilient force may preferably move the stack of measuring means units having the form of strips within the magazine unit.

According to a second aspect of the invention a container for loading a measuring means unit into a measuring device is provided. Said container is a container according to claim 6.

The second aspect of the invention is directed to a container according to claim 6 for loading a blood glucose level test strip into a blood glucose level measuring device. Said container comprises a casing defining an inner volume of the container, wherein the casing comprises a reception side for inserting at least one portion of a measuring device into the inner volume of the container, and wherein the casing is suited and adapted for storing a plurality of test strips, such that one of the test strips is retrievable within the inner volume of the container by means of a measuring device being inserted into the container through the reception side of the container.

Thus, the container is configured in such a way that the measuring device can be at least partially inserted into the container and such that it can retrieve within the container one of the measuring means units stored inside the container. In contrast to the prior art, the container is therefore not a dispenser, because it does not dispense a measuring means unit. According to the invention instead, the container is configured in such a way that the measuring means can protrude into the container and take out a measuring means unit.

Preferably, the container further comprises:
- a magazine unit for storing a plurality of measuring means units, wherein the magazine unit is located inside the casing;
- a first spring; and
- a holding means,
wherein the magazine unit is displaceable within the container against a resilient force of the first spring by inserting the at least one portion of a measuring device into the inner volume of the container, and wherein the holding means is configured to hold in a retrieval position exactly one of the measuring means units being located inside the container, such that said measuring means unit is not displaced during a displacing of the other measuring means units and such that it is retrievable in the retrieval position by means of the measuring device.

Thereby, the container is designed in a very simple and insusceptible way, such that the measuring device can protrude into the container and take out a measuring means unit. The magazine unit may comprise a cover to trap the measuring means units within the magazine unit. Such a cover may be opened only during manufacturing of the container to allow for a loading of measuring means units into the magazine unit. After loading, the cover may be irreversibly closed to trap the measuring means units within the magazine unit.

The first spring may be a separate or integrated part of the magazine unit. Preferably, the first spring is a flexible arm of a moulded plastic frame of the magazine unit, wherein said flexible arm abuts against the casing and deforms upon insertion of the at least one portion of the measuring device into the inner volume of the container, which results in the resilient force.

Preferably, the container comprises a second spring for shifting within the container the measuring means units being not retrieved, such that one of the measuring means units being not retrieved is shifted into the retrieval position. The second spring may be placed between two plates, which have essentially the same or a very similar shape or contour as the measuring means units. This has an advantage for the manufacturing process, because the magazine unit may be similarly loaded with the measuring means units and the pair of plates with the second spring inbetween. Preferably, the pair of plates with the second spring inbetween is installed after the magazine unit has been filled with a stack of measuring means units.

It is further advantageous if the resilient force of the second spring is directed essentially orthogonal with respect to the resilient force of the first spring.

In addition or as an alternative to the second spring, the magazine unit may move essentially orthogonal with respect to the resilient force of the first spring using a "sliding wedge" principle. Inclined faces at the magazine unit and the casing may correspond and slide on each other to force the magazine unit to move essentially orthogonal with respect to the insertion direction when the at least one portion of the measuring device is being inserted into the inner volume of the container. In order to ensure the uppermost measuring means units to be in the retrieval position, a certain path of the insertion process may be used for this before the displacement of the other measuring means units takes place.

The container is preferably separate from a measuring device, but may also be coupled to the measuring device by means of motion guidance, such as a rail. Such a coupling is preferably decoupable to allow for an easy exchange of the container.

The magazine unit is preferably configured to contain a stack of measuring means units having the form of strips, wherein the measuring means units are stackable into the direction of the resilient force of the second spring. Preferably, the measuring means units are a stack of blood glucose test strips for a blood glucose measuring device, said stack being storable in the magazine unit. For communicating the fill level of the magazine unit to the user, the container may comprise a display. For example, such a display may be a transparent portion of the casing through which the fill level is visible. A counter display or a display on a scale is also possible.

Preferably, the magazine unit is configured to be loaded with test strips of a length of less than 26 mm. Even more preferred is a length of less than 15 mm, wherein a length of less than 10 mm is most preferred. In this respect, it was shown that this is sufficient for a reliable measurement and that a manual handling of the test strips is not needed any more when the present invention is used.

In order to prevent the measuring means units from absorbing moisture, the container may comprise a desiccant. The desiccant may for example be a small package of desiccant disposed in the inner volume of the container. The material of the casing of the container may comprise at least partially a desiccant or be completely comprised of a desiccant.

The reception side of the container may comprise a closable opening for protection against moisture and contamination, wherein the opening allows inserting of at least one portion of the measuring device, but is closable or closed, respectively, after withdrawal. This opening may be a manually openable and closable lid, which tightly seals the casing as much as possible. However, alternatively an opening is conceivable which closes automatically by flexible and partially overlapping slats. In doing so, the slats are pressed inside into the container by the measuring device during inserting and deformed in such a way that at the least one portion of the measuring device can enter. During retrieving the measuring device, the flexible slats go back automatically into their overlapping position closing the opening.

The container comprises a plurality of measuring means units being stored inside the casing of the container. The casing of the container is closed in such a way that the measuring means units stored therein are not replaceable. Thereby, the container may be designed very simple and tight as a disposable article. Thus, there is no need any more for refilling of measuring means units, which may be unhygienic and sometimes combined with unintended moistening. In such an embodiment, the measuring means units are part of the container.

According to a third aspect of the invention, a kit according to claim 13 is provided.

The third aspect of the invention is a kit according to claim 13 inter alia comprising:
- a blood glucose level measuring device for measuring the blood glucose level of a person by using a blood glucose level test strip; and
- a container for loading a test strip into the measuring device, wherein the container is separate from the measuring device,
wherein the measuring device comprises an insertion portion, the insertion portion being equipped with a test strip slot
and configured for being inserted into an inner volume of a casing of the container through a reception side of the container in order to retrieve a test strip in the test strip slot, wherein the casing of the container defines the inner volume.

The container is a container according to the present invention as described above. The kit is used for loading the measuring device with measuring means units by the method according to the present invention as described above.

The measuring device and the container are preferably designed complementary to each other such that inserting of the insertion portion of the measuring device into the container is possible and the retrieval means of the measuring device within the container is positioned correctly for retrieval.

Preferably, the form of the insertion portion of the measuring device corresponds to the form of the reception side of the container in such a way that the insertion portion of the measuring device can only be inserted into the inner volume of the container when it has a defined orientation with respect to the container. This prevents incorrect convergence of the measuring device and the container. In case of a manual convergence, an accurate fit centring may be supported by an at least partially concave reception side of the container and a corresponding at least partially convex insertion portion of the measuring device. Alternatively, this may be achieved by corresponding inclined faces or corresponding conical faces.

Preferably, a measuring means unit can be jammed in the retrieving means by inserting the insertion portion of the measuring device into the inner volume of the container.

For example, the measuring means unit held in the retrieval position may be pressed into a tapering slot, and thereby jammed therein, during insertion by a preferably manually applied pressure. This may be supported by corresponding engaging means. A tapering slot, for instance, may have an inner wall pressing inwards by a resilient force. Such a wall may comprise contact pins, which touch contact surfaces of the measuring means unit in an electrically conductive manner by a resilient force. Thereby, the jamming for holding the measuring means unit provides simultaneously an electrically conductive contact for supplying a voltage to a sensor portion within the measuring means unit.

In case of a blood glucose level measuring device loaded with blood glucose level test strips, a sufficiently large area of the test strip must be accessible to be able to apply a drop of body fluid, such as blood or tears, thereon for the measurement. Preferably, an absorptive portion of the test strip protrudes out of the measuring device, on which a drop of body fluid may be applied, wherein the body fluid is transported through a capillary into a sensor portion, where it reacts by means of the glucose oxidase enzyme. One end of the test strip that is equipped with contact faces is preferably jammed into the measuring device such that the measuring device can supply through the contact faces a voltage in the sensor portion of the test strip, such that the measuring device can measure a current being characteristic for a blood glucose level by means of the body fluid having reacted by means of the glucose oxidase enzyme, and such that the measuring device can display the glucose concentration.

In order the dispose a read-out test strip in a hygienic way after measurement, the measuring device of the kit may comprise an actuable releasing means such that the read-out test strip can fall out of the measuring device without finger contact when the measuring device is held in a certain position. For instance, a resilient force jamming the test strip may be loosened by a manual actuating means. Alternatively, the releasing means may be an ejecting means, which actively ejects a read-out test-strip out of the measuring device after measurement by a manual actuating means.

In the following, the invention is discussed in further detail along the attached figures, wherein:
Figs. 1-5 show a perspective view of an embodiment of the kit according to the present invention, comprising a measuring device and a container, at different steps of a preferred embodiment of the method according to the present invention for loading the measuring device with a measuring means unit: converging (Fig. 1), inserting (Fig. 2), retracting (Fig. 3), measuring (Fig. 4), disposing (Fig. 5);
Fig. 6 shows a perspective view of an embodiment of the container according to the present invention with an opened lid;
Fig. 7 shows a cross section thorough a horizontal plane of the container shown in Fig. 6 with an opened lid;
Figs. 8 and 9 show a vertical longitudinal cross section along an axis of motion of a first advantageous embodiment of the kit according to the present invention, comprising a measuring device and a container, before inserting (Fig. 8) and with inserted measuring device after insertion (Fig. 9); and
Figs. 9 and 10 show a vertical longitudinal cross section along an axis of motion of a second advantageous embodiment of the kit according to the present invention, comprising a measuring device and a container, before inserting (Fig. 8) and with inserted measuring device after insertion (Fig. 9).

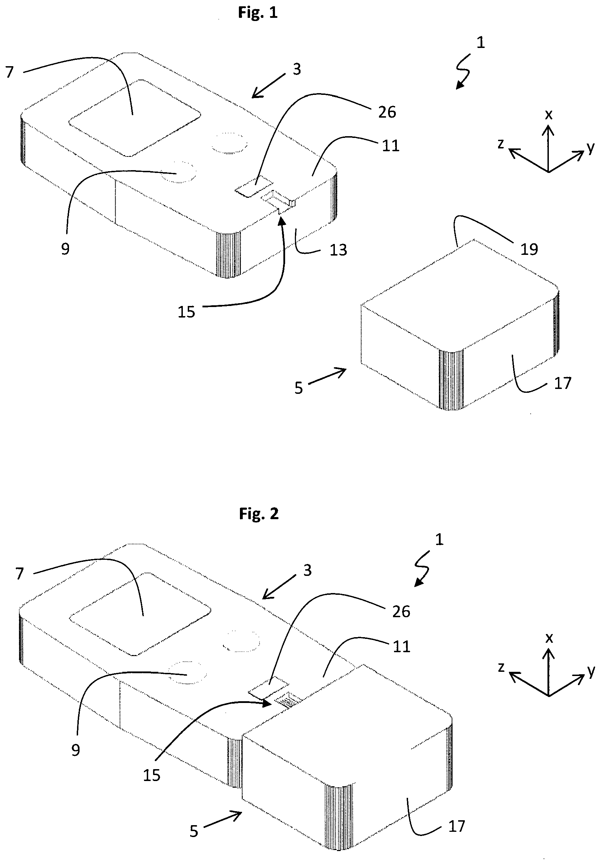

Figures 1 to 5 show a kit 1 comprising a measuring device 3 in form of a blood glucose level measuring device and a container 5 being separate from the measuring device 3. It is convenient to define a right-handed local Cartesian coordinate system that is shown in all figures and fixed to the container 5, because the measuring device 3 and the container 5 are freely movable with respect to each other. The z-axis follows the longitudinal axis of the container 5, the y-axis extends in the lateral direction and the x-axis is the vertical axis. Thereby, the direction of motion may be unambiguously specified with respect to the container 5. A direction of motion x of the container 5 itself with respect to unmoved objects display in the local coordinate system as a motion of those objects in the direction -*̅x̅*̅.

The measuring device 3 is substantially block-shaped with rounded vertical edges and ergonomically adapted for manual grip, such that the measuring device 3 can be comfortably held in one hand. On its upper side, the measuring device 3 comprises a display 7 for visually displaying the measurement result. Alternatively or in addition, a load speaker may be conceivable for acoustically signalling a measurement result in order to facilitate usage of the measuring device for blind or visually handicapped users. Furthermore, control elements 9 on form of push buttons are located on the surface, wherein a measurement may be started and/or stopped or other functions of the measuring device 3 may be activated and/or deactivated by said push buttons. The measuring device 3 itself may be equipped without a display 7 or control elements 9 but with a communication interface through which another device may control and read out the measuring device 3, such that displaying or signalling of the measuring result is possible on that other device.

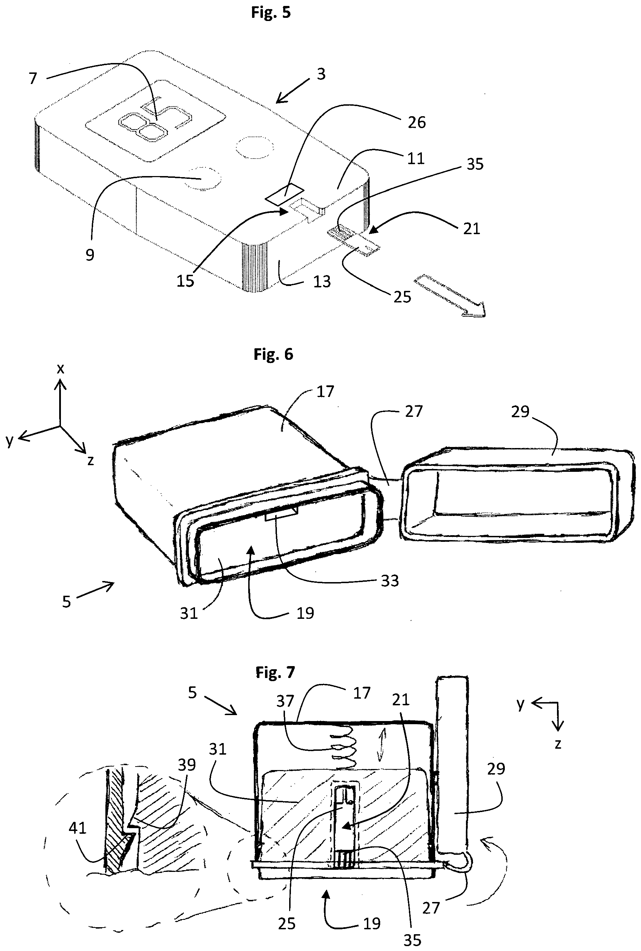

One portion of the measuring device 3 facing the container 5 may be called insertion portion 11, which can be inserted into the container 5. The insertion portion 11 of the measuring device 3 has an insertion side 13 facing the container 5, wherein a retrieval means 15 is located centrally at the insertion side's 13 upper edge. The container 5 comprises a casing 17 defining an inner volume of the container 5 with respect to the outside. The side of the container 5 facing the measuring device 3 is a reception side 19, the form of which corresponds to the form of the insertion portion 11 of the measuring device 3 (see Fig. 6) in such a way that the insertion portion 11 of the measuring device 3 can only be inserted into the inner volume of the container 5 when it has a defined orientation with respect to the container 5. As shown, this is the case when the vertical axis of the measuring device 3 extends into the x-direction, the cross axis of the measuring device 3 extends into the y-direction, and the insertion portion 11 of the measuring device 3 and the reception side 19 of the container 19 can be moved into the z-direction towards each other.

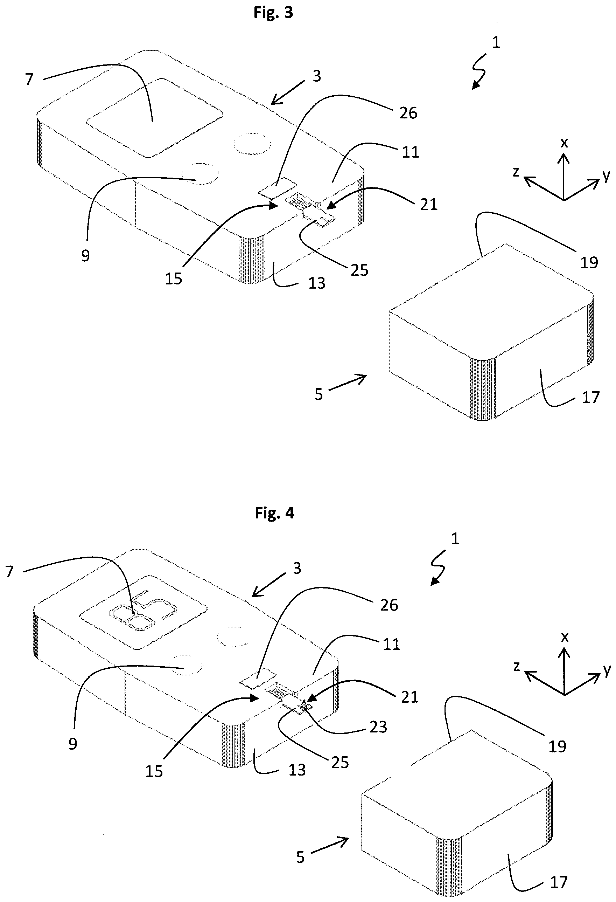

Fig. 1 shows a state during convergence of the measuring device 3 and the container 5 along the z-axis before the measuring device 3 and the container 5 touch each other. In Fig. 2, the measuring device 3 and the container 5 have contact already and the insertion portion 11 of the measuring device 3 is just being inserted into the container 5. In Fig. 3, the measuring device 3 is already again retracted out of the container 5 and, after retrieval of a measuring means unit 21 in form of a blood glucose level test strip, loaded with said measuring means unit 21 within the retrieval means 15. In Fig. 4, a drop of blood 23 is already applied to an absorptive portion 25 protruding out of the measuring device 3, and the measurement was performed. The measuring device 3 shows a value of 85 mg/dl on the display 7 corresponding to a molar glucose concentration of 4,72 mmol/l.

In Fig. 5, only the measuring device 3 is shown, wherein a releasing means comprising a button 26 was actuated, which loosened the measuring means unit 21 in the retrieval means 15 such that the used measuring means unit 21 may be "dumped out" after measurement without manual touch of the used measuring means unit 21 by adequate rotation of the measuring device 3. Alternatively, the releasing means may be configured as an active ejecting means, wherein a read-out test strip may be actively ejected out of the measuring device 3 upon an actuation of the releasing means.

Fig. 6 shows an embodiment of the container 5 according the present invention, wherein the container 5 comprises, at the reception portion 19, a lid 29 being hinged by a flexible connection 27 and opened. The lid 29 can, preferably airtightly, seal the inner volume of the container 5 defined by the casing 17 such that the inner volume is particularly protected against air humidity. The container 5 preferably comprises a desiccant such as silica gel in order to prevent the measuring means units located therein from absorbing humidity. The desiccant may, for instance, be provided in form of a small package of desiccant. Alternatively or in addition, the material of the casing 17 and/or of the lid 29 of the container 5 may comprises at least partially a desiccant or may be a desiccant.

A magazine unit 31 is located within the casing 17 of the container 5, wherein the outer surface of the magazine unit 31 is visible through the reception side 29 when the lid 29 is opened. At its outer surface, the magazine unit 31 comprises an opening slot 33 through which a measuring means unit 21 in form of a blood glucose level test strip fits lengthwise.

Fig. 7 shows a cross section thorough a horizontal plane of the container 5 as shown in Fig. 6 with the opened lid 29. There are measuring means units 21 stored which are stacked in x-direction within the magazine unit 31. The stored measuring means units 21 are blood glucose level test strips having a length of less than 15 mm, a width in y-direction of less than 5 mm and a thickness in x-direction of less than 0.5 mm. A new stack of measuring means units 21 comprises at least 20 test strips, wherein the uppermost test strip of the stack is positioned in a retrieval position directly behind the opening slot 33 of the magazine unit 31.

The measuring means units 21 each comprise a sensor portion 35 located at the end facing towards the opening slot 33 of the magazine unit 31. Moreover, the measuring means units 21 each comprise an absorptive portion 25 at the end facing away from the opening slot 33 of the magazine unit 31 for absorbing a drop of body fluid.

The magazine unit 31 is displaceable within the casing 17 into the negative z-direction against a first spring 37. Although the first spring 37 is shown as a helical spring, the first spring 37 may be a leaf spring or any other suitable spring means. As shown in the enlarged detail view, the magazine unit 31 is engaged with the casing 17 by an engaging means 39 such that the first spring 37 biases the magazine unit 31 against a stop 41. Thus, the stop 41 keeps the magazine unit 31, which is movable along the z-axis, within the casing 17. In this shown embodiment, the magazine unit 31 cannot be removed from the casing 17 and is therefore not refillable. Hence, the container 5 is a disposable article. Thus, there is no need any more for refilling of measuring means units 21, which may be unhygienic and sometimes combined with unintended moistening. In this embodiment, the measuring means units 21 are part of the container 5 according to the present invention.

Alternatively or in addition to the engaging means 39 and the stop 41, a separate fixation ring (not shown) may be used to trap the magazine unit 31 within the casing 17. During manufacturing of the container 5, such a fixation ring may be irreversibly clipped to the rim of the casing 17 at the reception side 19 after the magazine unit 31 equipped with a stack of measuring means units 21 has been installed.

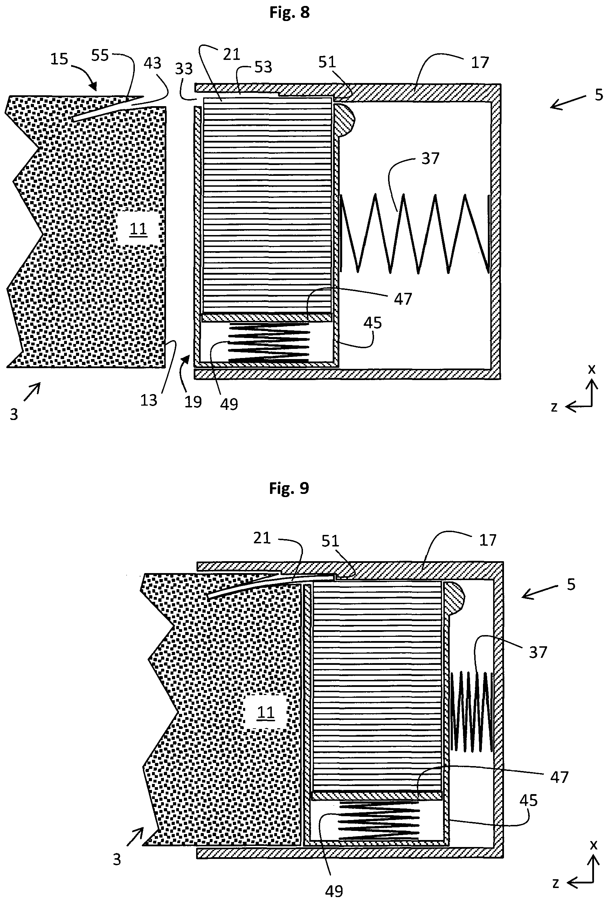

Fig. 8 shows a vertical longitudinal cut in the xz-plane through the container 5 shortly before the inserting of the measuring device 3. The measuring device 3 comprises an insertion portion 11, which has an insertion side 13 at the side facing towards the container 5. There is a retrieval means 15 at the upper edge of the insertion side 13. The retrieval means 15 comprises an insertion slot 43 through which a blood glucose level test strip can be inserted lengthwise. When the insertion side 13 is correctly positioned at the opened container 5 for loading the measuring device 3 with a measuring means unit 21, the insertion slot 43 is located directly at the opening slot 33 of the magazine unit 31 of the container 5

The measuring means units 21 are stacked in x-direction on a support plate 47, which is movable within a magazine frame 45, such that a second spring 49 being located below the support plate 47 biases upwards the support plate 47; and the stack of measuring means units 21 therewith. Although the second spring 49 is shown as a helical spring, the second spring 49 may be a leaf spring or any other suitable spring means. The second spring 49 abuts against the magazine frame 45 from below, wherein, within the casing 17, the magazine frame 45 is mounted movably in the negative z-direction against the first spring 37.

In the initial position shown in Fig. 8, the magazine unit 31 has a maximum position in z-direction. There is a holding means 51 in form of a stop edge located at the upper inner side of the casing 17, said stop edge engaging behind the uppermost measuring means unit 21 of the stack, said uppermost measuring means unit 21 being located in the retrieval position. Preferably, the holding means 51 extends into the inner volume of the container by 50% to 90% of the thickness of a measuring means unit 21. This ensures that the holding means 51 safely engages the uppermost measuring means unit 21, but not together with the second uppermost measuring means unit 21. The container 5 comprises a protruding space 53 above the uppermost measuring means unit 21 towards the reception side 19 in order to facilitate the retrieval of the uppermost measuring means unit 21, wherein said protruding space 53 preferably extends over the forward two thirds of the length of a measuring means unit 21 at most.

Fig. 9 shows a situation in which the measuring device 3 being inserted into the container 5 is retrieving the uppermost measuring means unit 21. The measuring device 3 was manually pressed with its insertion side 13 through the reception side 19 against the outer side of the magazine unit 31. The magazine unit 31 was displaced deeper into the casing 17 in the negative z-direction by manually holding the casing 17. In the end position shown in Fig. 9, the magazine unit 31 has a minimal position in z-direction, wherein the first spring 37 is maximally compressed. However, during the displacing, the uppermost measuring means unit 21 was held in the retrieval position. The opening slot 33 can move backwardly when the uppermost measuring means unit 21 is being held in the retrieval position, because the opening slot 33 of the magazine unit 31 extends at most about 10% to 50% of the thickness of one measuring means unit 21 below the uppermost measuring means unit 21. The uppermost measuring means unit 21 is therefore passively guided through the opening slot 33.

A tongue 55 of the retrieval means 15 of the measuring device 3 protrudes into the protruding space 53 during the inserting and guides the uppermost measuring means unit 21 into the insertion slot 43 of the retrieval means 15, said insertion slot 43 extending in an inclined way into the inner of the measuring device 3. Therein, the sensor portion 35 of the uppermost measuring means unit 21 is jammed such that the measuring device 3 can be retracted again out of the container 5 together with the jammed measuring means unit 21. During retracting of the measuring device 5 in z-direction, the magazine unit 31 follows in z-direction by means of the resilient force of the first spring 37. As soon as the magazine unit 31 has reached its maximal position in z-direction (as shown in Fig. 8), the second spring 49 shifts the still stored measuring means units 21 upwards by one position. The previously second uppermost measuring means unit 21 is the uppermost measuring means unit 21 after retrieval and shifts into the retrieval position.

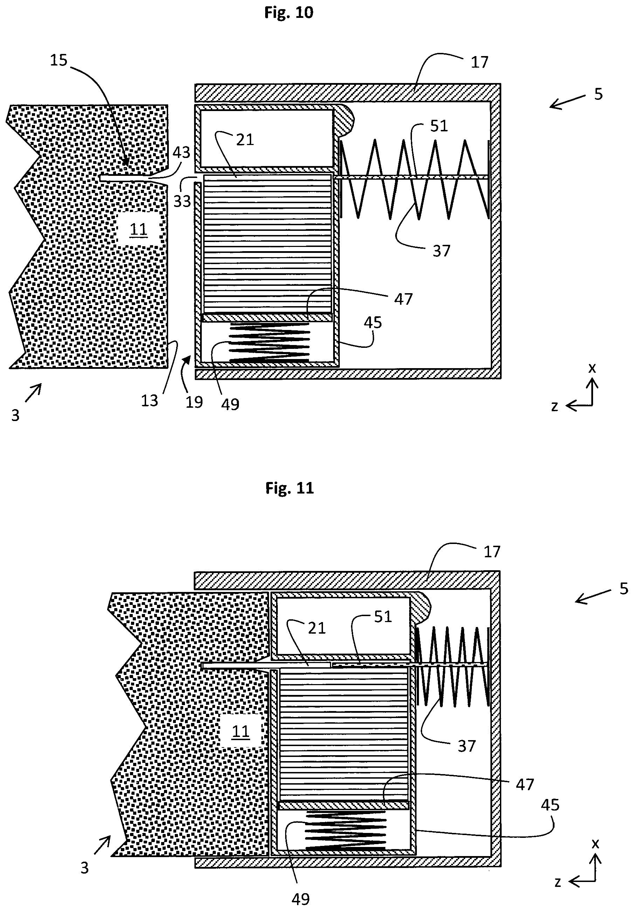

Figs. 10 and 11 show an alternative to the embodiment shown in Figs. 8 and 9. Therein, the retrieval position is not located at the casing 17, but with a distance to it. Accordingly, the opening slot 33 of the magazine unit 31 is located lower. This has the advantage that already available measuring devices with a correspondingly lower insertion slot 43 can be loaded with the container 5. However, it may be a disadvantage that the full height of the inner volume of the container 5 cannot be used as filling height for measuring means units 21.

A "passive pusher" being fixed with respect to the casing 17 and abutting against the casing 17 may serve as the holding means 51. The holding means 51 holds the uppermost measuring means unit 21 in the retrieval position when the other measuring means units 21 are being displaced backwardly. There is no need for a dedicated protruding space above the uppermost measuring means unit 21, because there is already enough space above the uppermost measuring means unit 21 due to the lower retrieval position.

With the present invention, a measuring device 3 can be loaded with a measuring means unit 21 solely by converging of the measuring device 3 and the container 5 in a remarkably simple, safe, hygienic and economic way.

### List of reference numerals:

- 1: kit
- 3: measuring device
- 5: container
- 7: display
- 9: control elements
- 11: insertion portion of the measuring device
- 13: insertion side of the measuring device
- 15: retrieval means of the measuring device
- 17: casing
- 19: reception side
- 21: measuring means unit
- 23: drop of blood
- 25: absorptive portion of the measuring means unit
- 26: button
- 27: flexible connection
- 29: lid
- 31: magazine unit
- 33: opening slot
- 35: sensor portion of the measuring means unit
- 37: first spring
- 39: engaging means
- 41: body stop
- 43: insertion slot
- 45: magazine frame
- 47: support plate
- 49: second spring
- 51: holding means
- 53: protruding space
- 55: tongue of the measuring device

## Claims

1. A method for loading a measuring device (3) with a measuring means unit (21) by using the kit of any of the claims 13 to 15 comprising the steps of:
- inserting at least one portion (11) of the measuring device (3) into a container (5), said container (5) comprising a plurality of measuring means units (21);
- retrieving one of the measuring means units (21) inside of the container (5) by means of the measuring device (3); and
- retracting at least one portion (11) of the measuring device (3) together with the retrieved measuring means unit (21) out of the container (5).

2. The method of claim 1, further comprising the steps of:
- displacing a plurality of measuring means units (21) within the container (5) by the step of inserting the at least one portion (11) of the measuring device (3) against a first resilient force,
wherein the measuring means units (21) are located inside the container (5); and
- holding in a retrieval position exactly one of the
measuring means units (21) being located inside the container (5), such that said measuring means unit (21) is not displaced (21) during the step of displacing of the other measuring means units (21) and such that it is retrieved in the retrieval position by means of the measuring device (3).

3. The method of claim 2, wherein further the displaced measuring means units (21) being located inside the container (5) are displaced back to an initial configuration within the container (5) by the first resilient force during the step of retracting the at least one portion (11) of the measuring device (3).

4. The method of claim 2 or 3, wherein further the displaced measuring means units (21) are shifted within the container (5) by a second resilient force, such that one of the measuring means units (21) not being retrieved is shifted into the retrieval position.

5. The method of claim 4, wherein the shifting is carried out essentially orthogonally with respect to the displacing.

6. A container (5) for loading a measuring means unit (21) into a measuring device (3), said container (5) comprising a casing (17) defining an inner volume of the container (5),
wherein the casing (17) comprises a reception side (19) for inserting at least one portion (11) of a measuring device (3) into the inner volume of the container (5), and
wherein the casing (17) is suited and adapted for storing a plurality of measuring means units (21), such that one of the measuring means units (21) is retrievable within the inner volume of the container (5) by means of a measuring device (3) being inserted into the container (5) through the reception side (19) of the container (5),
wherein the container (5) comprises a plurality of measuring means units (21) being located inside the casing (17) of the container (5), and wherein the casing (17) is closed, such that the measuring means units (21) stored therein cannot be replaced and
wherein the measuring units (21) are blood glucose level test strips and the measuring device (3) is a blood glucose level measuring device.

7. The container of claim 6, further comprising:
- a magazine unit (31) for storing a plurality of measuring means units (21), wherein the magazine unit (31) is located inside the casing (17);
- a first spring (37); and
- a holding means (51),
wherein the magazine unit (31) is displaceable within the container (5) against a resilient force of the first spring by inserting the at least one portion (11) of a measuring device (3) into the inner volume of the container (5), and
wherein the holding means is configured to hold in a retrieval position exactly one of the measuring means units (21) being located inside the container (5), such that said measuring means unit (21) is not displaced (21) during a displacing of the other measuring means units (21) and such that it is retrievable in the retrieval position by means of the measuring device (3).

8. The container of claim 7, further comprising:
- a second spring (49) for shifting within the container (5) the measuring means units (21) being not retrieved, such that one of the measuring means units (21) not being retrieved is shifted into the retrieval position.

9. The container of claim 8, wherein a resilient force of the second spring (49) is directed essentially orthogonal with respect to the resilient force of the first spring (37).

10. The container of any of the claims 6 to 9, wherein the container (5) is separate from a measuring device (3).

11. The container of any of the claims 8 to 10, wherein the magazine unit (31) is configured to contain a stack of measuring means units (21) having the form of strips, wherein the measuring means units (21) are stackable into the direction of the resilient force of the second spring (49).

12. The container of any of the claims 6 to 11, wherein the container (5) comprises a plurality of measuring means units (21) having the form of strips and being located inside the casing (17) of the container (5), wherein the measuring means units (21) are shorter than 27 mm, preferably shorter than 15 mm and ideally shorter than 10 mm.

13. A kit comprising
- a measuring device (3) for measuring by using a measuring means unit (21); and
- a container (5) for loading a measuring means unit (21) into the measuring device (3),
wherein the container (5) is separate from the measuring device (3), wherein the measuring device (3) comprises an insertion portion (11), the insertion portion (11) being equipped with a retrieving means (15) and configured for being inserted into an inner volume of a casing (17) of the container (5) through a reception side (19) of the
container (5) in order to retrieve a measuring means unit (21), wherein the casing (17) of the container (5) defines the inner volume, wherein the container (5) comprises a plurality of measuring means units (21) being located inside the casing (17) of the container (5), and wherein the casing (17) is closed, such that the measuring means units (21) stored therein cannot be replaced, and
wherein the measuring units (21) are blood glucose level test strips and the measuring device (3) is a blood glucose level measuring device.

14. The kit comprising of claim 13, wherein the form of the insertion portion (11) of the measuring device (3) corresponds to the form of the reception side (19) of the container (5) in such a way that the insertion portion (11) of the measuring device (3) can only be inserted into the inner volume of the container (5) when it has a defined orientation with respect to the container (5).

15. The kit of claim 13 or 14, wherein a measuring means unit (21) can be jammed in the retrieving means (15) by inserting the insertion portion (11) of the measuring device (3) into the inner volume of the container (5).

## Patentansprüche

1. Verfahren zum Beladen einer Messvorrichtung (3) mit einer Messmitteleinheit (21) unter Verwendung des Kits nach einem der Ansprüche 13 bis 15, wobei das Verfahren die folgenden Schritte umfasst:
- Einsetzen zumindest eines Abschnitts (11) der Messvorrichtung (3) in einen Behälter (5), wobei der Behälter (5) eine Vielzahl von Messmitteleinheiten (21) umfasst;
- Abrufen einer der Messmitteleinheiten (21) innerhalb des Behälters (5) mittels der Messvorrichtung (3); und
- Zurückziehen zumindest eines Abschnitts (11) der Messvorrichtung (3) zusammen mit der abgerufenen Messmitteleinheit (21) aus dem Behälter (5).

2. Verfahren nach Anspruch 1, des Weiteren umfassend die folgenden Schritte:
- Verschieben einer Vielzahl von Messmitteleinheiten (21) innerhalb des Behälters (5) durch den Schritt des Einsetzens des zumindest einen Abschnitts (11) der Messvorrichtung (3) gegen eine erste elastische Kraft,
wobei die Messmitteleinheiten (21) sich im Inneren des Behälters (5) befinden; und
- Halten genau einer der Messmitteleinheiten (21), die sich im Inneren des Behälters (5) befinden, in einer Abrufposition, so dass die Messmitteleinheit (21) während des Schritts des Verschiebens der anderen Messmitteleinheiten (21) nicht verschoben wird, und so, dass sie in der Abrufposition mittels der Messvorrichtung (3) abgerufen wird.

3. Verfahren nach Anspruch 2, wobei des Weiteren die verschobenen Messmitteleinheiten (21), die sich im Inneren des Behälters (5) befinden, durch die erste elastische Kraft während des Schritts des Zurückziehens des zumindest einen Abschnitts (11) der Messvorrichtung (3) zurück in eine Ausgangskonfiguration innerhalb des Behälters (5) verschoben werden.

4. Verfahren nach Anspruch 2 oder 3, wobei des Weiteren die verschobenen Messmitteleinheiten (21) innerhalb des Behälters (5) durch eine zweite elastische Kraft verlagert werden, so dass eine der Messmitteleinheiten (21), die nicht abgerufen werden, in die Abrufposition verlagert wird.

5. Verfahren nach Anspruch 4, wobei die Verlagerung im Wesentlichen orthogonal in Bezug auf die Verschiebung erfolgt.

6. Behälter (5) zum Laden einer Messmitteleinheit (21) in eine Messvorrichtung (3), wobei der Behälter (5) ein Gehäuse (17) umfasst, das ein Innenvolumen des Behälters (5) definiert,
wobei das Gehäuse (17) eine Aufnahmeseite (19) zur Einführung zumindest eines Abschnitts (11) einer Messvorrichtung (3) in das Innenvolumen des Behälters (5) umfasst, und
wobei das Gehäuse (17) dazu geeignet und angepasst ist, um eine Vielzahl von Messmitteleinheiten (21) zu speichern, so dass eine der Messmitteleinheiten (21) innerhalb des Innenvolumens des Behälters (5) abrufbar ist, mittels einer Messvorrichtung (3), die in den Behälter (5) durch die Aufnahmeseite (19) des Behälters (5) eingesetzt wird,
wobei der Behälter (5) eine Vielzahl von Messmitteleinheiten (21) umfasst, die sich im Inneren des Gehäuses (17) des Behälters (5) befinden, und
wobei das Gehäuse (17) geschlossen ist, so dass die darin gespeicherten Messmitteleinheiten (21) nicht ausgetauscht werden können, und
wobei die Messeinheiten (21) Blutzuckerspiegel-Teststreifen sind, und die Messvorrichtung (3) eine Blutzuckerspiegel-Messvorrichtung ist.

7. Behälter nach Anspruch 6, des Weiteren umfassend:
- eine Magazineinheit (31) zur Speicherung einer Vielzahl von Messmitteleinheiten (21), wobei die Magazineinheit (31) sich im Inneren des Gehäuses (17) befindet;
- eine erste Feder (37); und
- ein Haltemittel (51),
wobei die Magazineinheit (31) innerhalb des Behälters (5) gegen eine elastische Kraft der ersten Feder verschiebbar ist, indem der zumindest eine Abschnitt (11) einer Messvorrichtung (3) in das Innenvolumen des Behälters (5) eingesetzt wird, und wobei das Haltemittel dazu ausgestaltet ist, genau eine der Messmitteleinheiten (21), die sich im Inneren des Behälters (5) befinden, in einer Abrufposition zu halten, so dass die Messmitteleinheit (21) während einer Verschiebung der anderen Messmitteleinheiten (21) nicht verschoben wird, und so, dass sie in der Abrufposition mittels der Messvorrichtung (3) abrufbar ist.

8. Behälter nach Anspruch 7, des Weiteren umfassend:
- eine zweite Feder (49) zur Verlagerung der Messmitteleinheiten (21), die nicht abgerufen werden, innerhalb des Behälters (5), so dass eine der Messmitteleinheiten (21), die nicht abgerufen wurden, in die Abrufposition verlagert wird.

9. Behälter nach Anspruch 8, wobei eine elastische Kraft der zweiten Feder (49) im Wesentlichen orthogonal in Bezug auf die elastische Kraft der ersten Feder (37) gerichtet ist.

10. Behälter nach einem der Ansprüche 6 bis 9, wobei der Behälter (5) von einer Messvorrichtung (3) getrennt ist.

11. Behälter nach einem der Ansprüche 8 bis 10, wobei die Magazineinheit (31) dazu ausgestaltet ist, einen Stapel von Messmitteleinheiten (21) in der Form von Streifen zu enthalten, wobei die Messmitteleinheiten (21) in der Richtung der elastischen Kraft der zweiten Feder (49) stapelbar sind.

12. Behälter nach einem der Ansprüche 6 bis 11, wobei der Behälter (5) eine Vielzahl von Messmitteleinheiten (21) in der Form von Streifen umfasst und sich im Inneren des Gehäuses (17) des Behälters (5) befindet, wobei die Messmitteleinheiten (21) kürzer als 27 mm, vorzugsweise kürzer als 15 mm und idealerweise kürzer als 10 mm sind.

13. Kit, das Folgendes umfasst:
- eine Messvorrichtung (3) zur Messung unter Verwendung einer Messmitteleinheit (21); und
- einen Behälter (5) zum Laden einer Messmitteleinheit (21) in die Messvorrichtung (3),
wobei der Behälter (5) separat von der Messvorrichtung (3) ist,
wobei die Messvorrichtung (3) einen Einsetzabschnitt (11) umfasst, wobei der Einsetzabschnitt (11) mit einem Abrufmittel (15) ausgestattet und dazu ausgestaltet ist, durch eine Aufnahmeseite (19) des Behälters (5) in ein Innenvolumen eines Gehäuses (17) des Behälters (5) eingesetzt zu werden, um eine Messmitteleinheit (21) abzurufen, wobei
das Gehäuse (17) des Behälters (5) das Innenvolumen definiert,
wobei der Behälter (5) eine Vielzahl von Messmitteleinheiten (21) umfasst, die sich im Inneren des Gehäuses (17) des Behälters (5) befinden, und
wobei das Gehäuse (17) geschlossen ist, so dass die darin gespeicherten Messmitteleinheiten (21) nicht ausgetauscht werden können, und
wobei die Messeinheiten (21) Blutzuckerspiegel-Teststreifen sind, und die Messvorrichtung (3) eine Blutzuckerspiegel-Messvorrichtung ist.

14. Kit nach Anspruch 13, wobei die Form des Einsetzabschnitts (11) der Messvorrichtung (3) der Form der Aufnahmeseite (19) des Behälters (5) auf solche Weise entspricht, dass der Einsetzabschnitt (11) der Messvorrichtung (3) nur in das Innenvolumen des Behälters (5) eingesetzt werden kann, wenn er eine definierte Orientierung in Bezug auf den Behälter (5) aufweist.

15. Kit nach Anspruch 13 oder 14, wobei eine Messmitteleinheit (21) in das Abrufmittel (15) geklemmt werden kann, indem der Einsetzabschnitt (11) der Messvorrichtung (3) in das Innenvolumen des Behälters (5) eingesetzt wird.

## Revendications

1. Procédé pour charger un dispositif de mesure (3) avec une unité de moyen de mesure (21) en utilisant le kit selon l'une quelconque des revendications 13 à 15, le procédé comprenant les étapes suivantes :
- insérer au moins une portion (11) du dispositif de mesure (3) dans un récipient (5), ledit récipient (5) comprenant une pluralité d'unités de moyen de mesure (21) ;
- récupérer une des unités de moyen de mesure (21) à l'intérieur du récipient (5) au moyen du dispositif de mesure (3) ; et
- retirer du récipient (5) au moins une portion (11) du dispositif de mesure (3) ensemble avec l'unité de moyen de mesure (21) récupérée.

2. Procédé selon la revendication 1, en outre comprenant les étapes suivantes :
- déplacer une pluralité d'unités de moyen de mesure (21) dans le récipient (5) par l'étape d'insérer l'au moins une portion (11) du dispositif de mesure (3) contre une première force élastique,
les unités de moyen de mesure (21) étant disposées à l'intérieur du récipient (5) ; et
- fixer exactement une des unités de moyen de mesure (21) disposées à l'intérieur du récipient (5) dans une position de récupération, de telle manière que l'unité de moyen de mesure (21) n'est pas déplacée (21) pendant le déplacement des autres unités de moyen de mesure (21), et de telle manière qu'elle est récupérée dans la position de récupération au moyen du dispositif de mesure (3).

3. Procédé selon la revendication 2, dans lequel les unités de moyen de mesure (21) déplacées qui sont disposées à l'intérieur du récipient (5) sont déplacées vers leur configuration initiale dans le récipient (5) par la première force élastique, pendant l'étape de retirer l'au moins une portion (11) du dispositif de mesure (3).

4. Procédé selon la revendication 2 ou 3, dans lequel les unités de moyen de mesure (21) déplacées sont en outre décalées à l'intérieur du récipient (5) par une seconde force élastique, de telle manière qu'une des unités de moyen de mesure (21) pas récupérées est décalée dans la position de récupération.

5. Procédé selon la revendication 4, dans lequel le décalage est effectué sensiblement orthogonalement par rapport au déplacement.

6. Récipient (5) pour charger une unité de moyen de mesure (21) dans un dispositif de mesure (3), le récipient (5) comprenant un boîtier (17) définissant un volume intérieur du récipient (5),
dans lequel le boîtier (17) comporte une face de réception (19) pour insérer au moins une portion (11) d'un dispositif de mesure (3) dans le volume intérieur du récipient (5), et
dans lequel le boîtier (17) est conformé et adapté pour stocker une pluralité d'unités de moyen de mesure (21), de telle manière qu'une des unités de moyen de mesure (21) peut être récupérée dans le volume intérieur du récipient (5) au moyen d'un dispositif de mesure (3) inséré dans le récipient (5) à travers la face de réception (19) du récipient (5),
dans lequel le récipient (5) comprend une pluralité d'unités de moyen de mesure (21) disposées dans le boîtier (17) du récipient (5), et
dans lequel le boîtier (17) est fermé de telle manière que les unités de moyen de mesure (21) y stockées ne peuvent pas être remplacées, et
dans lequel les unités de mesure (21) sont des bandelettes de test de glycémie, et le dispositif de mesure (3) est un dispositif de mesure de glycémie.

7. Récipient selon la revendication 6, en outre comprenant :
- une unité de magasin (31) pour stocker une pluralité d'unités de moyen de mesure (21), l'unité de magasin (31) étant disposée dans le boîtier (17) ;
- un premier ressort (37) ; et
- un moyen de fixation (51),
l'unité de magasin (31) étant déplaçable à l'intérieur du récipient (5) contre une force élastique du premier ressort en insérant l'au moins une portion (11) d'un dispositif de mesure (3) dans le volume intérieur du récipient (5), et
le moyen de fixation étant configuré pour fixer exactement une des unités de moyen de mesure (21) disposées à l'intérieur du récipient (5) dans une position de récupération, de telle manière que l'unité de moyen de mesure (21) n'est pas déplacée (21) pendant le déplacement des autres unités de moyen de mesure (21), et de telle manière qu'elle peut être récupérée dans la position de récupération au moyen du dispositif de mesure (3).

8. Récipient selon la revendication 7, en outre comprenant :
- un second ressort (49) pour décaler, à l'intérieur du récipient (5), les unités de moyen de mesure (21) pas récupérées, de telle manière qu'une des unités de moyen de mesure (21) pas récupérée est décalée vers la position de récupération.

9. Récipient selon la revendication 8, dans lequel une force élastique du second ressort (49) est orientée sensiblement orthogonalement par rapport à la force élastique du premier ressort (37).

10. Récipient selon l'une quelconque des revendications 6 à 9, le récipient (5) étant séparé d'un dispositif de mesure (3).

11. Récipient selon l'une quelconque des revendications 8 à 10, dans lequel l'unité de magasin (31) est configurée pour contenir une pile d'unités de moyen de mesure (21) sous forme de bandelettes, les unités de moyen de mesure (21) étant empilables dans la direction de la force élastique du second ressort (49).

12. Récipient selon l'une quelconque des revendications 6 à 11, le récipient (5) comprenant une pluralité d'unités de moyen de mesure (21) sous forme de bandelettes, disposées dans le boîtier (17) du récipient (5), les unités de moyen de mesure (21) étant plus courtes que 27 mm, de préférence plus courtes que 15 mm, et idéalement plus courtes que 10 mm.

13. Kit comprenant
- un dispositif de mesure (3) pour effectuer une mesure en utilisant une unité de moyen de mesure (21) ; et
- un récipient (5) pour charger une unité de moyen de mesure (21) dans le dispositif de mesure (3),
dans lequel le récipient (5) est séparé du dispositif de mesure (3), le dispositif de mesure (3) comprenant une portion d'insertion (11), la portion d'insertion (11) étant munie d'un moyen de récupération (15) et étant configurée à être insérée dans un volume intérieur d'un boîtier (17) du récipient (5) à travers d'une face de réception (19) du récipient (5), afin de récupérer une unité de moyen de mesure (21), le boîtier (17) du récipient (5) définissant le volume intérieur,
dans lequel le récipient (5) comprend une pluralité d'unités de moyen de mesure (21) disposées dans le boîtier (17) du récipient (5), et
dans lequel le boîtier (17) est fermé de telle manière que les unités de moyen de mesure (21) y stockées ne peuvent pas être remplacées, et
dans lequel les unités de mesure (21) sont des bandelettes de test de glycémie, et le dispositif de mesure (3) est un dispositif de mesure de glycémie.

14. Kit selon la revendication 13, dans lequel la forme de la portion d'insertion (11) du dispositif de mesure (3) correspond à la forme de la face de réception (19) du récipient (5) de telle manière que la portion d'insertion (11) du dispositif de mesure (3) ne peut être insérée dans le volume intérieur du récipient (5) qu'avec une orientation définie par rapport au récipient (5).

15. Kit selon la revendication 13 ou 14, dans lequel une unité de moyen de mesure (21) peut être coincée dans le moyen de récupération (15) en insérant la portion d'insertion (11) du dispositif de mesure (3) dans le volume intérieur du récipient (5).
